(19) **Europäisches Patentamt**

**European Patent Office**

**Office européen des brevets**

(11) **EP 1 002 529 A1**

## (12) EUROPEAN PATENT APPLICATION

(43) Date of publication:
24.05.2000 Bulletin 2000/21

(51) Int. Cl.7: **A61K 9/16**, A61K 9/50

(21) Application number: 99203867.9

(22) Date of filing: 06.09.1995

(84) Designated Contracting States:
**AT BE CH DE DK ES FR GB GR IE IT LI LU MC NL PT SE**
Designated Extension States:
**LT LV SI**

(30) Priority: 09.09.1994 JP 21644994
14.12.1994 JP 31029194

(62) Document number(s) of the earlier application(s) in accordance with Art. 76 EPC:
**95930707.5 / 0 779 806**

(71) Applicant:
**Takeda Chemical Industries, Ltd.
Osaka-shi, Osaka 541-0045 (JP)**

(72) Inventors:
• **Igari, Yasutaka
Kobe-shi, Kyogo 658 (JP)**
• **Yamagata, Yutaka
Hyogo 654 (JP)**
• **Iinuma, Satoshi
Hyogo 654 (JP)**
• **Okada, Hiroaki
Osaka 565 (JP)**

(74) Representative:
**Lewin, John Harvey et al
Elkington and Fife,
Prospect House,
8 Pembroke Road
Sevenoaks, Kent TN13 1XR (GB)**

(54) **Sustained-release preparation containing a metal salt of a peptide**

(57)     A sustained-release preparation containing a water-insoluble or slightly water-soluble polyvalent metal salt of a water-soluble peptide type of physiologically active substance except for an endothelin antagonist and a biodegradable polymer.

The present invention is a sustained-release preparation that is highly efficient in incorporating a water-soluble peptide type of physiologically active substance except for an endothelin antagonist and suppresses initial burst of the water-soluble peptide type of physiologically active substance except for an endothelin antagonist. The sustained-release preparation of the present invention is capable of releasing the water-soluble peptide type of physiologically active substance except for an endothelin antagonist while retaining its bioactivity after administration *in vivo*. Furthermore, the water-soluble peptide type of physiologically active substance except for an endothelin antagonist in the sustained-release preparation is kept stable for a long period of time, with little loss of bioactivity.

## Description

Technical Field

The present invention relates to a sustained-release preparation which comprises a water-insoluble or a slightly water-soluble polyvalent metal salt of a water-soluble peptide type of physiologically active substance except for an endothelin antagonist and a biodegradable polymer.

Background Art

Physiologically active substances, particularly peptides or derivatives thereof, are known to exhibit various pharmacologic actions *in vivo*. Some have been produced in large amounts, for pharmaceutical application, by chemical synthesis, or as a result of advances in gene engineering and cell engineering technologies, using organisms such as *Escherichia coli*, yeasts, animal cells and hamsters. However, these peptides must be administered frequently, since they generally have a short biological half-life, and so pose a significant physical burden of injection on patients. To solve this problem, various attempts have been made to develop sustained-release preparations.

The first problem to solve in developing a sustained-release preparation of a water-soluble physiologically active substance, particularly a water-soluble peptide (hereinafter also referred to as "peptide") is to control peptide solubility, i.e., to regulate the peptide release rate.

Japanese Publication of the Translation of International Patent Application No. 500286/1991 discloses an insoluble zinc-protamine-α-interferon complex.

Japanese Patent Unexamined Publication No. 2930/1988 discloses a system comprising a polylactide in which a macromolecular polypeptide is dispersed.

Japanese Patent Unexamined Publication Nos. 221855/1993 and 172208/1994 disclose a technology by which a water-soluble peptide is converted to a water-insoluble peptide salt, which is then suspended in an organic medium containing a biodegradable polymer to efficiently incorporate the water-soluble peptide in fine grains. The water-insoluble peptide used in these patent publications is an organic acid salt formed at the base portion of the water-soluble peptide molecule, and is exemplified by pamoate, tannic acid, stearic acid or palmitate.

Although there have been various attempts to produce sustained-release preparations of water-soluble physiologically active substances, as stated above, no satisfactory sustained-release preparations have been obtained; there is therefore need for the development of a sustained-release preparation that is highly efficient in incorporating water-soluble physiologically active substance, suppresses initial water-soluble physiologically active substance burst, offers a constant water-soluble physiologically active substance release rate, and keeps the bioactivity of water-soluble physiologically active substance.

Disclosure of Invention

Through extensive investigation to solve the above problems, the present inventors found that a sustained-release preparation, having dramatically increased efficiency of water-soluble peptide type of physiologically active substance except for an endothelin antagonist incorporation in a biodegradable polymer and showing little drug burst just after administration to the living body, can be obtained by producing a water-insoluble or a slightly water-soluble polyvalent metal salt of a water-soluble peptide type of physiologically active substance except for an endothelin antagonist (hereinafter also referred to as "complex"), which salt is formed from a combination of a water-soluble peptide type of physiologically active substance except for an endothelin antagonist having an acidic group, or a water-soluble salt thereof (hereinafter also referred to as "physiologically active substance"), with a water-soluble polyvalent metal salt, and dispersing or dissolving it in a biodegradable polymer. After further investigations based on this finding, the inventors developed the present invention.

Accordingly, the present invention relates to:

(1) a sustained-release preparation which comprises

    (a) a water-insoluble or slightly water-soluble polyvalent metal salt of a water-soluble peptide type of physiologically active substance except for an endothelin antagonist and
    (b) a biodegradable polymer,

(2) a preparation of term 1 above, wherein the physiologically active substance is a water-soluble peptide or a derivative thereof,
(3) a preparation of term 2 above, wherein the peptide is a hormone, cytokine, hematopoietic factor, growth factor, enzyme, soluble or solubilized receptor, antibody, antigen containing peptide, blood coagulation factor or adhesion molecule,

(4) a preparation of term 2 above, wherein the peptide is a hormone,

(5) a preparation of term 4 above, wherein the hormone is a growth hormone

(6) a preparation of term 3 above, wherein the hormone is an insulin,

(7) a preparation of term 2 above, wherein the peptide is a cytokine,

(8) a preparation of term 7 above, wherein the cytokine is an interferon,

(9) a preparation of term 2 above, wherein the peptide is a growth factor,

(10) a preparation of term 1 above, wherein the polyvalent metal salt is a transition metal salt,

(11) a preparation of term 1 above, wherein the polyvalent metal salt is a zinc salt,

(12) a preparation of term 1 above, wherein the solubility of the polyvalent metal salt to water is about 0 to about 0.1% (w/w) at 20°C,

(13) a preparation of term 1 above, wherein the solubility of the polyvalent metal salt to water is about 0 to about 0.01% (w/w),

(14) a preparation of term 1 above, which contains about 0.1 to about 50% (w/w) of the polyvalent metal salt,

(15) a preparation of term 1 above, which contains about 1 to about 30% (w/w) of the polyvalent metal salt,

(16) a preparation of term 1 above, wherein the biodegradable polymer is an aliphatic polyester,

(17) a preparation of term 16 above, wherein the aliphatic polyester is a polymer of lactic acid and glycolic acid,

(18) a preparation of term 17 above, wherein the composition ratio of lactic acid and glycolic acid is 100/0 to about 40/60 (mole%),

(19) a preparation of term 18 above, wherein the composition ratio is about 90/10 to about 45/55 (mole%),

(20) a preparation of term 17 above, wherein the weight-average molecular weight of the polymer is about 3,000 to about 20,000,

(21) a preparation of term 17 above, wherein the weight-average molecular weight of the polymer is about 3,000 to about 14,000,

(22) a preparation of term 16 above, wherein the alihatic polyester is a homopolymer of lactic acid,

(23) a preparation of term 22 above, wherein the weight-average molecular weight of the homopolymer is about 3,000 to about 20,000,

(24) a preparation of term 22 above, wherein a weight-average molecular weight of the homopolymer is about 3,000 to about 14,000,

(25) a preparation of term 1 above, wherein the preparation is a microcapsule,

(26) a preparation of term 25 above, wherein the microcapsule is for injection,

(27) a preparation of term 1 above, which is an injectable one,

(28) Use of a water-insoluble or slightly water-soluble polyvalent metal salt of a water-soluble peptide type of physiologically active substance except for an endothelin antagonist and a biodegradable polymer for the production of a sustained-release preparation, and

(29) a method of producing a sustained-release preparation, which comprises dispersing a water-insoluble or slightly water-soluble polyvalent metal salt of a water-soluble peptide type of physiologically active substance except for an endothelin antagonist in an oil phase containing a biodegradable polymer to make a solid-in-oil emulsion, adding the solid-in-oil emulsion to a water phase to make a solid-in-oil-in-water emulsion, and then in-water drying the soild-in-oil-in-water emulsion.

Incidentally abbreviations of amino acid, peptide or the like used in the present invention are based on those in accordance with IUPAC-IUB Commission on Biochemical Nomenclature or those conventionally used in the relevant fields, and possible optical isomers of amino acid are, unless otherwise specified, L-isomers.

The physiologically active substance in the water-insoluble or the slightly water-soluble polyvalent metal salt is a physiologically active substance having an acidic group. Here, the acidic group is exemplified by the carboxyl group and sulfo group. The physiologically active substance is preferably a physiologically active substance having a peptide bond or an amino acid and acidic group. The acidic group may be derived from an amino acid. More preferably, the physiologically active substance is a water-soluble peptide having an acidic group or a derivative thereof. A solubility of the physiologically active substance to water is 1% (w/w) or more at 25°C.

The physiologically active substance preferably has two or more carboxyl groups.

The molecular weight of the physiologically active substance is about 200 to 200,000, preferably about 200 to about 50,000, more preferably about 500 to about 40,000.

A representative activity of a physiologically active substance is hormone action. The physiologically active substance may be a natural, synthetic, semi-synthetic or genetically engineered product, or a derivative thereof. As concerns the mechanism of action, these physiologically active substances may be agonistic or antagonistic.

Physiologically active substances, particularly water-soluble peptide or a derivative thereof for the present invention include hormones, cytokines, hematopoietic factors, growth factors, enzymes, a soluble or solubilized receptor, an antibody or a fragment thereof, an antigen containing peptide, a blood coagulation factor, an adhesion molecule, agonists or antagonists capable of binding to receptors of the physiologically active substances and so on.

Example hormones include insulin, growth hormone, natriuretic peptide, gastrin, prolactin, adrenocorticotropic hormone (ACTH), thyroid-stimulating hormone (TSH), luteinizing hormone (LH), follicle-stimulating hormone (FSH), human chorionic gonadotropin (HCG), motilin, kallikrein and so on. The hormone is preferably insulin and growth hormone.

Example cytokines include lymphokines, monokines and so on. Example lymphokines include interferons (alpha, beta, gamma), interleukins (IL-2 through IL-12) and so on. Example monokines include an interleukin 1(IL-1), tumor necrosis factor and so on. The cytokine is preferably a lymphokine, more preferably an interferon (alpha, beta, gamma).

Example hematopoietic factors include erythropoietin, granulocyte colony-stimulating factor (G-CSF), macrophage colony-stimulating factor (M-CSF), thrombopoietin, platelet growth-stimulating factor, megakaryocyte potentiator and so on.

Example growth factors include basic or acidic fibroblast growth factors (FGF), members of the family thereof (e.g., FGF-9 etc.), nerve cell growth factor (NGF) or members of the family thereof, insulin-like growth factors (e.g., IGF-1, IGF-2), bone morphogenetic protein (BMP) or members of the family thereof and so on.

Example enzymes include superoxide dismutase (SOD), tissue plasminogen activator (TPA) and so on.

Example soluble receptors include soluble IL-6 receptor, insulin-like growth factor binding protein (IGFBP), soluble TNF receptor, soluble EGF receptor, soluble IL-1 receptor and so on.

Example solubilized receptors include a known receptors such as IL-1 receptor, IL-6 receptor, TNF receptor or Fas ligand etc., which is solubilized by a method of gene engineering.

Example antibodies include a human monoclonal antibody, a human-mouse chimeric monoclonal antibody in which the variable region of an antibody derived from mouse is bound to the constant region of an antibody derived from human, or a fragment thereof and so on. Example type of antibody include IgM, IgG, IgE and so on. Example antigenes, which is recognized by the above described antibody, include platelet, virus and so on.

Example blood coagulation factors include factor VIII and so on.

Example adhesion molecules include fibronectin, ICAM-1 and so on.

Furthermore, example physiologically active substances include endothelin, Arg-Gly-Asp-Ser (RGDS), pituitary adenylate cyclase activating polypeptide (PACAP) and so on.

The physiologically active substance is converted to a water-insoluble or a slightly water-soluble polyvalent metal salt thereof by bringing it into contact with a water-soluble polyvalent metal.

The polyvalent metal in the water-soluble polyvalent metal salt is exemplified by divalent, trivalent or tetravalent metal etc. such as alkaline earth metals (e.g., calcium, magnesium etc.), transition metals [e.g., iron (II, III), copper (II), zinc (II) etc.), the group $III_b$ metals [e.g., aluminum (II, III) etc.], the group $IV_b$ metals [e.g., tin (II, IV) etc.] and so on. The polyvalent metal is preferably alkaline earth metals or transition metals, more preferably calcium or zinc, still more preferably zinc.

Water-soluble polyvalent metal salts include salts of polyvalent metals and acids, e.g., salts of polyvalent metals and inorganic acids, and salts of polyvalent metals and organic acids.

The salt of a polyvalent metal and an acid is preferably a salt whose water solubility at normal temperature (20°C) is not lower than about 20 mg/ml, more preferably not lower than about 100 mg/ml, and still more preferably not lower than about 200 mg/ml.

Inorganic acids to form salts with polyvalent metals include hydrochloric acid, sulfuric acid, nitric acid, thiocyanic acid and so on.

Organic acids to form salts with polyvalent metals include aliphatic carboxylic acids and aromatic acids. The aliphatic carboxylic acid is preferably an aliphatic carboxylic acid having 2 to 9 carbon atoms. Aliphatic carboxylic acids include aliphatic monocarboxylic acids, aliphatic dicarboxylic acids, aliphatic tricarboxylic acids and so on. These aliphatic carboxylic acids may be saturated or unsaturated one.

Example aliphatic monocarboxylic acids include saturated aliphatic monocarboxylic acids having 2 to 9 carbon atoms (e.g., acetic acid, propionic acid, butyric acid, valeric acid, caproic acid, enanthic acid, caprylic acid, pelargonic acid, caprynic acid etc.) and unsaturated aliphatic monocarboxylic acids having 2 to 9 carbon atoms (e.g., acrylic acid, propiolic acid, methacrylic acid, crotonic acid, isocrotonic acid etc.).

Example aliphatic dicarboxylic acids include saturated aliphatic dicarboxylic acids having 2 to 9 carbon atoms (e.g., malonic acid, succinic acid, glutaric acid, adipic acid, pimelic acid etc.) and unsaturated aliphatic dicarboxylic acids having 2 to 9 carbon atoms (e.g., maleic acid, fumaric acid, citraconic acid, mesaconic acid etc.).

Example aliphatic tricarboxylic acids include saturated aliphatic tricarboxylic acids having 2 to 9 carbon atoms (e.g., tricarballylic acid, 1,2,3-butanetricarboxylic acid etc.).

The above-mentioned aliphatic carboxylic acids may have 1 or 2 hydroxyl groups. Such aliphatic carboxylic acids include glycolic acid, lactic acid, glyceric acid, tartronic acid, malic acid, tartaric acid, citric acid and so on.

The aliphatic carboxylic acid is preferably an aliphatic monocarboxylic acid, more preferably an aliphatic monocarboxylic acid having 2 to 9 carbon atoms, and still more preferably a saturated aliphatic monocarboxylic acid having 2 or 3 carbon atoms. Examples of particularly preferable aliphatic carboxylic acids include acetic acid and so on.

Example aromatic acids include benzoic acid, salicylic acid and so on, with preference given to benzoic acid.

Examples of salts of polyvalent metals and inorganic acids, i.e., inorganic acid polyvalent metal salts, include halides (e.g., zinc chloride, calcium chloride), sulfates, nitrates, thiocyanates and so on.

Examples of salts of polyvalent metals and aliphatic carboxylic acids, i.e., aliphatic carboxylic acid polyvalent metal salts, include calcium acetate, zinc acetate, calcium propionate, zinc glycolate, calcium lactate, zinc lactate, zinc tartrate and so on. Preferable aliphatic carboxylic acid polyvalent metal salts include calcium acetate and zinc acetate. Greater preference is given to zinc acetate and so on.

Examples of salts of polyvalent metals and aromatic acids, i.e., aromatic acid polyvalent metal salts, include benzoates, salicylates and so on. Greater preference is given to zinc benzoate.

A water-insoluble or a slightly water-soluble polyvalent metal salt of a physiologically active substance is produced by mixing in a solvent the water-soluble physiologically active substance and a water-soluble polyvalent metal salt. The mixing procedure is preferably conducted in water.

The mixing ratio (mole ratio) of the physiologically active substance and water-soluble polyvalent metal salt in water is, for example 1:1 to 1:1000, preferably 1:1 to 1:100, more preferably 1:1 to 1:50, still more preferably 1:1 to 1:10. The concentrations of both components in water may be optional, as long as they exceed the solubility of the resulting complex, within their respective solubility ranges.

The pH of the aqueous solution resulting from the above mixing must be such that the bioactivity of the physiologically active substance is not affected, and that the solubilities of the physiologically active substance and water-soluble polyvalent metal salt are not lowered in excess. Although the mixing procedure is normally conducted in distilled water, it may be conducted in water adjusted to weakly acidic, neutral, or weakly alkaline pH as necessary.

"Being water insoluble or slightly water-soluble" as mentioned herein is not irreversible but reversible, meaning that water solubility is very low. Water solubility is about 0 to about 0.1% (w/w), preferably about 0 to about 0.01% (w/w) at ordinary temperature (20°C).

The thus-obtained water insoluble or slightly water-soluble polyvalent metal salt of a water-soluble physiologically active substance is used after being vacuum dried or freeze dried as necessary.

In the sustained-release preparation of the present invention, the content of the water-insoluble or slightly water-soluble polyvalent metal salt of the physiologically active substance is normally about 0.1 to about 50% (w/w), preferably about 1 to about 30% (w/w).

The biodegradable polymer is exemplified by high-molecular polymers slightly soluble or insoluble in water, such as aliphatic polyesters (e.g., homopolymers, copolymers or mixtures thereof synthesized from one or more α-hydroxycarboxylic acids such as glycolic acid, lactic acid, hydroxybutyric acid etc.), hydroxydicarboxylic acids such as malic acid etc., hydroxytricarboxylic acids such as citric acid etc. and others, poly-α-cyanoacrylic acid esters, polyamino acids such as poly-γ-benzyl-L-glutamic acid and so on. These may be used in mixture at appropriate ratios. The type of polymerization may be random, block or graft.

The biodegradable polymer is preferably an aliphatic polyester (e.g., a homopolymer, copolymer or mixture thereof synthesized from one or more α-hydroxycarboxylic acids such as glycolic acid, lactic acid, hydroxybutyric acid etc., hydroxydicarboxylic acids such as malic acid etc., hydroxytricarboxylic acids such as citric acid etc. and others.

Of the above-mentioned aliphatic polyesters, homopolymers or copolymers synthesized from one or more α-hydroxycarboxylic acids (e.g., glycolic acid, lactic acid, hydroxybutyric acid etc.) are preferred from the viewpoint of reliable biodegradability and biocompatibility. More preferably, the aliphatic polyester is a copolymer synthesized from one or more α-hydroxycarboxylic acids (e.g., glycolic acid, lactic acid, hydroxybutyric acid etc.). Also, these copolymers may be used in mixture.

The biodegradable polymer for the present invention is produced by a commonly known method.

Although the above-described α-hydroxycarboxylic acid may be of the D-, L- or D,L-configuration, it is preferable that the ratio of the D-/L-configuration (mole%) fall within the range from about 75/25 to about 25/75. The ratio of the D-/L-configuration (mole%) is more preferably about 60/40 to about 30/70.

Example copolymers of the above-described α-hydroxycarboxylic acid include copolymers of glycolic acid with another α-hydroxy acid, which is preferably lactic acid or 2-hydroxybutyric acid.

The α-hydroxycarboxylic acid copolymer is preferably a lactic acid-glycolic acid copolymer or a 2-hydroxybutyric acid-glycolic acid copolymer.

More preferably, the α-hydroxycarboxylic acid copolymer is a lactic acid-glycolic acid copolymer.

With respect to the lactic acid-glycolic acid copolymer, it is preferable that the content ratio (lactic acid/glycolic acid) (mole%) be about 100/0 to about 40/60. The content ratio is more preferably about 90/10 to about 45/55, and more preferably about 80/20 to about 45/55. The weight-average molecular weight of the lactic acid-glycolic acid copolymer is about 3,000 to about 20,000, preferably about 3,000 to about 14,000 more preferably about 3,000 to about 12,000.

Also, the degree of dispersion of the lactic acid-glycolic acid copolymer (weight-average molecular weight/number-average molecular weight) is preferably about 1.2 to about 4.0, more preferably about 1.5 to about 3.5.

The lactic acid-glycolic acid copolymer can be synthesized by a known process, such as the method described in Japanese Patent Unexamined Publication No. 28521/1986. It is preferable that the copolymer be synthesized by catalyst-free dehydration polymerization condensation.

With respect to the 2-hydroxybutyric acid-glycolic acid copolymer, it is preferable that glycolic acid account for about 10 to about 75 mole% and 2-hydroxybutyric acid for the remaining portion. More preferably, glycolic acid accounts for about 20 to about 75 mole%, and still more preferably about 30 to about 70 mole%. The weight-average molecular weight of the 2-hydroxybutyric acid-glycolic acid copolymer is preferably about 2,000 to about 20,000. The degree of dispersion of the 2-hydroxybutyric acid-glycolic acid copolymer (weight-average molecular weight/number-average molecular weight) is preferably about 1.2 to 4.0, more preferably about 1.5 to 3.5. A 2-hydroxybutyric acid-glycolic acid copolymer can be synthesized by a known process, such as that described in Japanese Patent Unexamined Publication No. 28521/1986. It is preferable that the copolymer be synthesized by catalyst-free dehydration polymerization condensation.

Preferable example homopolymers of the above-described $\alpha$-hydroxycarboxylic acid include homopolymer of lactic acid. The weight-average molecular weight of the homopolymer of lactic acid is about 3,000 to about 20,000, preferably about 3,000 to about 14,000. A homopolymer of lactic acid can be synthesized by a known process, such as that described in Japanese Patent Unexamined Publication No. 28521/1986. It is preferable that the homopolymer be synthesized by catalyst-free dehydration polymerization condensation.

The above-described 2-hydroxybutyric acid-glycolic acid copolymer may be used in a mixture with polylactic acid. Although the polylactic acid may be of the D- or L-configuration or a mixture thereof, it is preferable that the ratio of the D-/L-configuration (mole%) fall within the range from about 75/25 to about 20/80. The ratio of the D-/L-configuration (mole%) is more preferably about 60/40 to about 25/75, and still more preferably about 55/45 to about 25/75. The weight-average molecular weight of polylactic acid is preferably about 1,500 to about 20,000, more preferably about 1,500 to 10,000. Also, the degree of dispersion of the polylactic acid is preferably about 1.2 to 4.0, more preferably about 1.5 to 3.5.

For producing polylactic acid, two methods are known: ring-opening polymerization of lactide, a dimer of lactic acid, and dehydration polymerization condensation of lactic acid. For obtaining a polylactic acid of relatively low molecular weight for the present invention, direct dehydration polymerization condensation of lactic acid is preferred. This method is, for example, described in Japanese Patent Unexamined Publication No. 28521/1986.

When a 2-hydroxybutyric acid-glycolic acid copolymer and polylactic acid are used in mixture, their mixing ratio is about 10/90 to about 90/10 (% by weight). The mixing ratio is preferably about 20/80 to 80/20, and more preferably about 30/70 to 70/30.

In the present specification, weight-average molecular weight is defined as the molecular weight obtained by gel permeation chromatography (GPC) with 9 polystyrenes as reference substances with respective weight-average molecular weights of 120,000, 52,000, 22,000, 9,200, 5,050, 2,950, 1,050, 580 and 162. Number-average molecular weight based on GPC measurement is also calculated. The degree of dispersion is calculated from the weight-average molecular weight and the number-average molecular weight. Measurements were taken using a GPC column KF804L × 2 (produced by Showa Denko) and an RI monitor L-3300 (produced by Hitachi, Ltd.) with chloroform as the mobile phase.

The above-described copolymer, synthesized by catalyst-free dehydration polymerization condensation, usually has a terminal carboxyl group.

In the present invention, the biodegradable polymer preferably has a terminal carboxyl group.

A biodegradable polymer having a terminal carboxyl group is a polymer in which the number-average molecular weight by GPC determination and that by terminal group determination almost agree.

By terminal group quantitation, number-average molecular weight is calculated as follows:

About 1 to 3 g of the biodegradable polymer is dissolved in a mixed solvent of acetone (25 ml) and methanol (5 ml), and the solution is quickly titrated with a 0.05 N alcoholic solution of potassium hydroxide while being stirred at room temperature with phenolphthalein as an indicator to determine the terminal carboxyl group content; the number-average molecular weight based on terminal group quantitation is calculated using the following equation:

Number-average molecular weight based on terminal group quantitation = 20,000 A/B

A: Weight mass (g) of the biodegradable polymer
B: Amount (ml) of the 0.05 N alcoholic solution of potassium hydroxide added until the titration end point is reached

For example, in the case of a polymer having a terminal carboxyl group, and synthesized from one or more α-hydroxy acids by catalyst-free dehydration polymerization condensation, the number-average molecular weight based on GPC measurement and the number-average molecular weight based on terminal group quantitation almost agree. On the other hand, in the case of a polymer having essentially no terminal carboxyl group, and synthesized from a cyclic dimer by ring-opening polymerization using a catalyst, the number-average molecular weight based on terminal group quantitation is significantly higher than the number-average molecular weight based on GPC determination. This difference makes it possible to clearly differentiate a polymer having a terminal carboxyl group from a polymer having no terminal carboxyl group.

While the number-average molecular weight based on terminal group quantitation is an absolute value, the number-average molecular weight based on GPC determination is a relative value that varies depending on various analytical conditions (e.g., kind of mobile phase, kind of column, reference substance, slice width chosen, baseline chosen etc.); it is therefore difficult to have an absolute numerical representation of the latter. However, the fact that the number-average molecular weight based on GPC determination and that based on terminal group quantitation almost agree means that the number-average molecular weight based on terminal group quantitation falls within the range from about 0.5 to about 2 times, preferably from about 0.8 to about 1.5 times, the number-average molecular weight based on GPC determination. Also, the fact that the number-average molecular weight based on terminal group quantitation is significantly higher than that based on GPC determination means that the number-average molecular weight based on terminal group quantitation is about 2 times or more the number-average molecular weight based on GPC determination.

The sustained-release preparation of the present invention is produced by dispersing in a biodegradable polymer a water-insoluble or a slightly water-soluble polyvalent metal salt of a physiologically active substance obtained by mixing the physiologically active substance and a water-soluble polyvalent metal salt. Methods of producing a sustained-release preparation include the in-water drying method, phase separation method, spray drying method, and modifications thereof.

Methods of producing a sustained-release preparation, e.g., microcapsules, are described below.

(i) In-water drying method (o/w method)

In this method, a solution of a biodegradable polymer in an organic solvent is first prepared. The organic solvent used to produce the sustained-release preparation of the present invention preferably has a boiling point not higher than 120°C. Such organic solvents include halogenated hydrocarbons (e.g., dichloromethane, chloroform, carbon tetrachloride etc.), alcohols (e.g., ethanol, methanol), acetonitrile and so on. These may be used in mixture at appropriate ratios. For example, when a dichloromethane and alcohols are used in mixture, their mixing ratio (v/v) is about 1000/1 to about 1/1, preferably about 100/1 to about 1/1, still more preferably about 10/1 to about 2/1. The organic solvent is preferably dichloromethane and acetonitrile, and still more preferably dichloromethane. The concentration of the biodegradable polymer in the organic solvent solution is normally about 0.01 to about 80% (w/w), preferably about 0.1 to about 70% (w/w), and more preferably about 1 to about 60% (w/w), depending on the molecular weight of the biodegradable polymer, kind of organic solvent and so on.

To the organic solvent solution of the biodegradable polymer thus obtained, a water-insoluble or a slightly water-soluble polyvalent metal salt of a physiologically active substance is added or dissolved, after being freeze-dried or vacuum dried as necessary. In this operation, the amount of complex added is set so that the complex:biodegradable polymer weight ratio is up to about 1:2, preferably about 1:3.

The organic solvent solution thus prepared is added to an aqueous phase to form an o/w emulsion using a turbine type mechanical stirrer or the like, followed by evaporation of the solvent in the oil phase, to yield microcapsules. The volume of the aqueous phase is normally chosen over the range of about 1 to about 10,000 times, preferably about 2 to about 5,000 times, and more preferably about 5 to about 2,000 times, the volume of the oil phase.

An emulsifier may be added to the external aqueous phase. The emulsifier may be any one, as long as it is capable of forming a stable o/w emulsion. Examples of such emulsifiers include anionic surfactants, nonionic surfactants, polyoxyethylene castor oil derivatives, polyvinylpyrrolidone, polyvinyl alcohol, carboxymethyl cellulose, lecithin, gelatin, hyaluronic acid and so on. These may be used in combination as appropriate. The emulsifier concentration in the external aqueous phase is preferably about 0.001 to about 20% (w/w), more preferably about 0.01 - about 10% (w/w), and still more preferably about 0.05 - about 5% (w/w).

In the above-described o/w method, microcapsules may be produced by a method in which the complex is dispersed in an organic solvent solution of a biodegradable polymer, i.e., the s/o/w method.

(ii) In-water drying method (w/o/w method)

In this method, a solution of a biodegradable polymer in an organic solvent is first prepared. The concentration of the biodegradable polymer in the organic solvent solution is normally about 0.01 to about 80% (w/w), preferably about 0.1 to about 70% (w/w), and more preferably about 1 to about 60%, depending on the molecular weight of the biodegradable polymer, kind of organic solvent and so on. An aqueous dispersion of the complex is used as the internal aqueous phase. The concentration of the complex in the aqueous dispersion is, for example, about 10 to about 90% (w/v). The above-described aqueous dispersion of the complex is emulsified and dispersed in the organic solvent solution of the biodegradable polymer to form a w/o emulsion by known methods of dispersion using a turbine type mechanical stirrer, homogenizer and so on. This operation is conducted in such a way as to bring the weight ratio of the internal aqueous phase and the biodegradable polymer up to about 1:2, preferably about 1:3. The ratio of the internal aqueous phase and the organic solvent solution of the biodegradable polymer is 1:1,000 to 1:1 (v/v), preferably 1:100 to 1:5 (v/v), and more preferably 1:50 to 1:5 (v/v).

The w/o emulsion thus prepared is then added to another aqueous phase to form a w/o/w emulsion, followed by evaporation of the solvent in the oil phase, to yield microcapsules. This operation is conducted in accordance with term (i) above.

The sustained-release preparation of the present invention is preferably used in the form of fine particles. This is because sustained-release preparation does not cause undue pain to the patient when administered via an injection needle for ordinary subcutaneous or intramuscular injection. The mean particle diameter of the sustained-release preparation, for example, is about 0.1 to about 300 μm, preferably about 1 to about 150 μm, and more preferably about 2 to about 100 μm.

In the present specification, a sustained-release preparation in fine particle form is also referred to as a microcapsule.

As used herein the term "microcapsule" may be referred to as "microsphere".

The sustained-release preparation of the present invention can, for example, be administered as microcapsules as such, or in the form of various dosage forms of non-oral preparations (e.g., intramuscular, subcutaneous or visceral injections or indwellable preparations, nasal, rectal or uterine transmucosal preparations etc.) or oral preparations (e.g., capsules such as hard capsules, soft capsules etc., solid preparations such as granules and powders etc., liquid preparations such as suspensions etc.).

In the present invention, the sustained-release preparation is preferably used for injection. When the sustained-release preparation is a microcapsule, for instance, it can be prepared as an aqueous suspension by suspending microcapsules in water, along with a dispersing agent (e.g., surfactants such as Tween 80 and HCO-60, polysaccharides such as carboxymethyl cellulose, sodium alginate and sodium hyaluronate etc.), a preservative (e.g., methyl paraben, propyl paraben etc.), an isotonizing agent (e.g., sodium chloride, mannitol, sorbitol, glucose etc.), etc., to yield a sustained-release preparation for injection of practical use. Alternatively, the sustained-release preparation of the present invention is prepared as an oily suspension by dispersing microcapsules, along with a vegetable oil such as sesame oil or corn oil with or without a phospholipid such as lecithin, or a medium-chain fatty acid triglyceride (e.g., MIGLYOL 812), to yield a sustained-release preparation for injection of practical use.

When the sustained-release preparation is a microcapsule, for instance, its mean particle size is chosen over the range from about 0.1 to about 300 μm as long as the requirements concerning degree of dispersion and needle passage are met, when it is to be used as an injectable suspension. Preferably, the particle size falls within the range from about 1 to about 150 μm, more preferably about 2 to about 100 μm.

The above-described microcapsule can be prepared as a sterile preparation, without limitation by the method in which the entire production process is sterile, the method in which gamma rays is used as sterilant, and the method in which an antiseptic is added.

With low toxicity, the sustained-release preparation of the present invention can be safely used in mammals (e.g., humans, bovines, swines, dogs, cats, mice, rats, rabbits etc.).

Indications for the sustained-release preparation of the present invention vary according to the physiologically active substance used. For example, the sustained-release preparation of the present invention is effective in the treatment or prevention of diabetes mellitus etc. when the physiologically active substance is insulin; renal cancer, hepatitis C etc. when the physiologically active substance is interferon alpha; anemia etc. when the physiologically active substance is erythropoietin; developmental failure when the physiologically active substance is growth hormone, and neutropenia etc. after anticancer chemotherapy when the physiologically active substance is granulocyte colony-stimulating factor. When the physiologically active substance is erythropoietin, the sustained-release preparation of the present invention is also effective in promoting hematopoiesis for autotransfusion.

Depending on the type and content of the physiologically active substance, duration of physiologically active substance release, target disease, subject animal and other factors, the dose of the sustained-release preparation may be set at levels such that the physiologically active substance exhibits its action. The dose per administration of the physiologically active substance is chosen as appropriate over the range from about 0.0001 to about 10 mg/kg body weight for each adult, when the preparation is a 1-week preparation. More preferably, the dose may be chosen as appropriate over the range about about 0.0005 to about 1 mg/kg body weight.

The dose per administration of the sustained-release preparation is preferably chosen as appropriate over the range from about 0.0005 to about 50 mg/kg body weight for each adult. More preferably, the dose is chosen as appropriate over the range from about 0.0025 to about 10 mg/kg body weight. Dosing frequency can be chosen as appropriate, e.g., once weekly, once every two weeks or once every four weeks, depending on type, content and dosage form of the physiologically active substance, duration of physiologically active substance release, subject disease, subject animal and other factors.

Although the preparation of the present invention may be stored at normal temperature or in a cold place, it is preferable to store it in a cold place. Normal temperature and a cold place as mentioned herein are as defined by the Pharmacopoeia of Japan, specifically, 15 to 25°C for normal temperatures and under 15°C for cold places.

Best Mode for Carrying Out the Invention

The present invention is hereinafter described in more detail by means of the following examples, which are not to be construed as limitative.

Reference Example 1

A solution of 0.5 g of swine insulin (27.3 U/mg, DIOSYNTH, Netherlands) in 22 ml of 100 mM sodium hydroxide aqueous solution and a solution of 1 g of zinc acetate (dihydrate) in 10 ml of distilled water were mixed together and kept standing at room temperature for 1 hour. After centrifugation at about 3,000 rpm (05PR-22, Hitachi, Ltd.), the supernatant was discarded. The residue was again dispersed in distilled water and centrifuged. After the supernatant was discarded, a small amount of distilled water was added to the residue, which was then freeze-dried to yield about 1 g of crude zinc salt of swine insulin as a dry powder.

To determine the insulin content in the powder thus obtained, the powder was extracted with a 50 mM EDTA solution containing 30% acetonitrile being shaken for 3 hours, followed by quantitation by high performance liquid chromatography (HPLC). It was shown that 47.6 mg of swine insulin was contained per 100 mg of dry powder.

Reference Example 2

To a mixture of 168 ml of 40% aqueous solution of potassium hydroxide and 1,000 ml of ethyl ether, 104 g of nitrosoethylurea was added little by little, while the mixture was stirred under ice cooling conditions. The resulting yellow ether layer was separated and dried by the addition of granular potassium hydroxide. The potassium hydroxide was then removed to yield about 900 ml of a diazoethane solution.

130 g of a lactic acid-glycolic acid copolymer (lactic acid/glycolic acid = 50/50 (mole%) of about 5,800 in weight-average molecular weight) was dissolved in 1,900 ml of methylene chloride, stirred and cooled. Under ice cooling conditions, the above diazoethane solution was added drop by drop, followed by stirring at room temperature for 2 hours. After the mixture was kept standing overnight, the solvent was distilled off under reduced pressure; the residue was vacuum dried at room temperature to yield 131 g of the ethyl ester of the lactic acid-glycolic acid copolymer.

Reference Example 3

A solution of 1 mg of human growth hormone (Biotechnology General, USA) in 0.9 ml of distilled water and a solution of 9.98, 29.43, 49.88, 69.84, 79.81 or 99.77 μg of zinc acetate (dihydrate) in 0.1 ml of distilled water were mixed together. A molar ratio of zinc atom to growth hormone are 1, 3, 5, 7, 8 and 10. In case of the molar ratio being 5, about 60 % of the human growth hormone was precipitated. In case of the molar ratio being 7 or more, almost 100% of the human growth hormone was precipitated.

Reference Example 4

1 g of leuprolide acetate (TAP-144) and 157.5 mg of gelatin were dissolved in 1 ml of distilled water at 70 to 80°C. To the aqueous solution warming at the temperature being slightly higher than the gelation temperature of the aqueous solution, 21 g of solution of lactic acid-glycolic acid copolymer, which was prepared by dissolving 7.85 g of the lactic acid-glycolic acid copolymer [lactic aicd/glycolic acid: 75/25 (mole %), viscosity: 0.142 to 0.169 cP] in 13.15 g of dichloromethane, was added. The mixture was emulsified with a compact homogenizer for several minutes or more to provide a W/O emulsion. The obtained W/O emulsion was cooled to 10 to 20°C. The emulsion was poured in 5000 ml of 0.1% (w/v) aqueous polyvinyl alcohol solution which temperature was adjusted to 10 to 20°C and the mixture was emulsified using a turbine homomixer to provide a W/O/W emulsion. This W/O/W emulsion was stirred at room temperature (15 to 30°C) to evaporate the dichloromethane and, thereby, solidify the internal W/O emulsion, after which the microcapsules were collected by centrifugation. These microcapsules were redispersed in distilled water and further centrifuged to wash off the excess drug and polyvinyl alcohol. The recovered microcapsules were suspended in a small amount of distilled water. To the suspension, 1.5 g of D-mannitol was added and dissolved. The obtained suspension was freeze-dried under reduced pressure to provide powdery microcapsules.

After lyophilization, the obtained microcapsules as a powder were further dried at 50°C under reduced pressure, viz. a temperature 3°C higher than Tmg of the matrix component lactic acid-glycolic acid copolymer, for 24, 48, 96 or 120 hours to provide powdery sustained-release microcapsules.

Example 1

To 200 ml of an aqueous solution of interferon alpha (containing 40 billion IU), 1 ml of an aqueous solution of zinc acetate (dihydrate) (200 mg/ml) and 1 ml of 1 N sodium hydroxide were added; after mixing, the mixture was kept standing at 4°C overnight. After centrifugation at 3,000 rpm, the insoluble complex was recovered and freeze-dried to yield about 200 mg of crude zinc salt of interferon alpha.

To a solution of 1.5 g of a lactic acid-glycolic acid copolymer (lactic acid/glycolic acid ratio = 50/50, molecular weight 5,800, produced by Wako Pure Chemical Industries) and 1.5 g of the ethyl ester of lactic acid-glycolic acid copolymer obtained in Reference Example 2 in 4 ml of dichloromethane, 200 mg of the above-described crude zinc salt of interferon alpha was added, followed by stirring for about 30 seconds using a homogenizer (Polytron) to yield an s/o emulsion. This emulsion was poured in 700 ml of a 0.1% (w/w) aqueous solution of polyvinyl alcohol (EG-40, produced by The Nippon Synthetic Chemical Industry) previously adjusted to 18°C, followed by stirring in a turbine homomixer at 6,000 rpm to yield an s/o/w emulsion. This emulsion was stirred at room temperature for 3 hours to volatilize the dichloromethane and solidify the oil phase. Subsequently, after centrifugation at about 2,000 rpm (05PR-22, Hitachi, Ltd.), the supernatant was discarded. The resulting residue was again dispersed in distilled water and centrifuged. After the collected microcapsules were re-dispersed in a small amount of distilled water in the presence of 50 mg of D-mannitol, the dispersion was freeze-dried to yield powder microcapsules.

Example 2

3.6 g of a lactic acid-glycolic acid copolymer (lactic acid/glycolic acid = 75/25 (mole%), weight-average molecular weight 13,585, number-average molecular weight 4,413, produced by Wako Pure Chemical Industries) was dissolved in 6.6 g (5 ml) of dichloromethane. 420 mg of the crude zinc salt of swine insulin obtained in Reference Example 1 (containing 200 mg of swine insulin) was dispersed in 6.6 g (5 ml) of dichloromethane. Both were mixed and stirred for about 10 seconds in a homogenizer (Polytron) to yield an s/o emulsion. This emulsion was poured in 800 ml of a 0.1% (w/w) aqueous solution of polyvinyl alcohol (EG-40, produced by The Nippon Synthetic Chemical Industry), previously adjusted to 18°C, followed by stirring in a turbine homomixer at 6,000 rpm to yield an s/o/w emulsion. This emulsion was then stirred at room temperature for 3 hours to volatilize the dichloromethane and solidify the oil phase. After centrifugation at about 2,000 rpm (05PR-22, Hitachi, Ltd.), the supernatant was discarded. The residue was again dispersed in distilled water and centrifuged. After the collected microcapsules were re-dispersed in a small amount of distilled water in the presence of 50 mg of D-mannitol, the dispersion was freeze-dried to yield powder microcapsules (about 3 g recovered).

To determine the insulin content in the microcapsules thus obtained, the powder was extracted by shaking with a 50 mM EDTA solution containing 30% acetonitrile for 3 hours, followed by quantitation by high performance liquid chromatography (HPLC). It was shown that 6.2 mg of insulin was contained per 100 mg of microcapsules.

Example 3

To 8 ml of an erythropoietin injection solution (Espo™ Injection 3000, produced by Sankyo) (containing 12,000 IU), 1 g of zinc chloride was added little by little; the mixture was kept standing at room temperature for 1 hour. After the mixture was centrifuged at 3,000 rpm, the precipitate was again dispersed in distilled water and centrifuged to yield a precipitate. To this precipitate, a small amount of distilled water was added, followed by freeze-drying, to yield 60 mg of a mixture of crude zinc salt of erythropoietin and crude zinc salt of albumin as a powder.

To a solution of 0.5 g of a lactic acid-glycolic acid copolymer (lactic acid/glycolic acid ratio = 50/50, molecular weight 14,000, produced by Wako Pure Chemical Industries) in 1.5 ml of dichloromethane, 60 mg of the above-described mixture of crude zinc salt of erythropoietin and crude zinc salt of albumin was added, followed by stirring for about 30 seconds using a homogenizer (Polytron), to yield an s/o emulsion. This emulsion was then treated in the same manner as in Example 1 to yield 152 mg of powder microcapsules.

Example 4

Human growth hormone (Genotropin™ 16IU, produced by Sumitomo Pharmaceuticals) was dissolved in 1 ml of distilled water. To this solution, 100 μl of an aqueous solution of zinc chloride (10 mg/ml) was added; the mixture was kept standing at room temperature for 1 hour. The mixture was then centrifuged; the precipitate was again dispersed in distilled water and centrifuged to yield a precipitate. To this precipitate, a small amount of distilled water was added, followed by freeze-drying, to yield 5.6 mg of crude zinc salt of human growth hormone as a powder.

To a solution of 0.5 g of a lactic acid-glycolic acid copolymer (lactic acid/glycolic acid ratio = 75/25, molecular weight 9,800, produced by Wako Pure Chemical Industries) in 1.5 ml of dichloromethane, 5.6 mg of the above-described crude zinc salt of human growth hormone was added, followed by stirring for about 30 seconds using a homogenizer (Polytron), to yield an s/o emulsion. This emulsion was then treated in the same manner as in Example 1 to yield 121 mg of powder microcapsules.

Example 5

After 10 ml (containing $3 \times 10^8$ IU) of a granulocyte colony-stimulating factor (G-CSF) injection solution [Filgrastin Neupogen, trade name, Amgen, USA) was neutralized with a dilute aqueous solution of sodium hydroxide, 1 ml of an aqueous solution of zinc chloride (10 mg/ml) was added; the mixture was kept standing at room temperature for 1 hour. The mixture was then centrifuged; the precipitate was again dispersed in distilled water and centrifuged to yield a precipitate. To this precipitate, a small amount of distilled water was added, followed by freeze-drying, to yield 4 mg of crude zinc salt of granulocyte colony-stimulating factor as a powder.

To a solution of 0.5 g of a lactic acid-glycolic acid copolymer (lactic acid/glycolic acid ratio = 50/50, molecular weight 8,000, produced by Wako Pure Chemical Industries) in 1.5 ml of dichloromethane, 4 mg of the above-described crude zinc salt of granulocyte colony stimulating factor was added, followed by stirring for about 30 seconds using a homogenizer (Polytron), to yield an s/o emulsion. This emulsion was then treated in the same manner as in Example 1 to yield 110 mg of powder microcapsules.

Example 6

After 5.21 mg (26 U/mg) of human insulin (human recombinant insulin, purchased from Wako Pure Chemical Industries) was dissolved in 0.63 ml of a 57 mM aqueous solution of hydrochloric acid, 0.35 ml of a 0.05 N aqueous solution of sodium hydroxide was added to yield a human insulin solution of nearly neutral pH. To this human insulin solution, 0.2 ml of an aqueous solution of zinc acetate (20 mg/ml) was added; the mixture was kept standing at 4°C overnight. The mixture was then centrifuged at about 3,000 rpm; the precipitate was again dispersed in distilled water and centrifuged to yield a precipitate. To this precipitate, a small amount of distilled water was added, followed by freeze-drying, to yield 11 mg of crude zinc salt of human insulin as a powder.

To a solution of 0.5 g of a lactic acid-glycolic acid copolymer (lactic acid/glycolic acid ratio = 50/50, molecular weight 6,000, produced by Wako Pure Chemical Industries) in 1.5 ml of dichloromethane, 11 mg of the above-described crude zinc salt of human insulin was added, followed by stirring for about 30 seconds using a homogenizer (Polytron), to yield an s/o emulsion. This emulsion was then treated in the same manner as in Example 1 to yield 105 mg of powder microcapsules.

Comparative Example

To a solution of 0.9 g of lactic acid-glycolic acid copolymer [lactic acid/glycolic acid ratio=50/50 (mole%), weight-average molecular weight 6,000, produced by Wako Pure Chemical Industries] in 1.5 ml of dichloromethane, 100 mg of a substantially zinc free human insulin [zinc content being under 0.0001% (w/w)] was added, followed by stirring for about 10 seconds using a homogenizer (Polytron), to yield an s/o emulsion. This emulsion was then treated in the same manner as in Example 1 to yield powder microcapsules (470 mg).

To determine the insulin content in the microcapsules thus obtained, the powder was extracted by shaking with a 50 mM EDTA solution containing acetonitrile for 3 hours, followed by quantitation by high performance liquid chromatography (HPLC). It was shown that 8.7 mg of insulin was contained per 100 mg of microcapsules.

Experimental Example 1

323 mg of powder microcapsules as obtained in Example 2 was dispersed in a 1 ml of dispersant for injection (5 mg of carboxymethyl cellulose, 1 mg of polysorbate 80 and 50 mg of mannitol dissolved per ml distilled water). The resulting dispersion was subcutaneously administered to the backs of 6-week-old male SD rats (insulin administered at about 20 mg per rat). After administration, blood was collected via the tail at constant intervals and assayed for serum swine insulin concentration using an enzyme immunoassay (EIA) kit (produced by Sanko Junyaku). Active swine insulin was detected in serum for 1 week or more after administration.

Experimental Example 2

70 mg of powder microcapsules as obtained in Example 4 was dispersed in a 0.5 ml of dispersant for injection (5 g of carboxymethyl cellulose, 2 g of polysorbate 80 and 50 g of mannitol dissolved per liter distilled water). The resulting dispersion was subcutaneously administered to the backs of 6-week-old male SD rats (growth hormone administered at about 3 mg per rat). After administration, blood was collected via the tail at constant intervals and assayed for serum growth hormone concentration by radio immunoassay. Active growth hormone was detected in serum for 1 week or more after administration.

Comparative Experimental Example

154.7 mg of powder microcapsule as obtained in Comparative Example was dispersed in a 1.75 ml of dispersant for injection (5 mg of carboxymethyl cellulose, 1 mg of polysorbate 80 and 50 mg of mannitol dissolved per ml distilled water). The resulting dispersion was subcutaneously administered to the backs of 6-week-old male SD rats (insulin administered at about 44 mg per rat). After administration, blood was collected via the tail at constant intervals and assayed for serum insulin concentration by an enzyme immunoassay (EIA). Active insulin was detected in serum only at 1 day after administration.

Industrial Applicability

According to the present invention, it is possible to provide a sustained-release preparation that is highly efficient in incorporating physiologically active substance and suppresses initial physiologically active substance burst. The sustained-release preparation of the present invention is capable of releasing the physiologically active substance while retaining its bioactivity after administration *in vivo*. Furthermore, the physiologically active substance in the sustained-release preparation is kept stable for a long period of time, with little loss of bioactivity.

**Claims**

1.   A sustained-release preparation which comprises

(a) a water-insoluble or slightly water-soluble polyvalent metal salt of a water-soluble peptide type of physiologically active substance except for an endothelin antagonist wherein the solubility in water is 0.01 to 0.1 % (w/w) at 20°C, and
(b) a biodegradable polymer,
in which the content of the water-insoluble or slightly water-soluble polyvalent metal salt of the physiologically active substance in the sustained-release preparation is 0.1 to 50% (W/W).

2.   A preparation according to claim 1, wherein the physiologically active substance is a water-soluble peptide or a derivative thereof.

3.   A preparation according to claim 2, wherein the peptide is a hormone, cytokine, hematopoietic factor, growth factor, enzyme, soluble or solubilized receptor, antibody, antigen containing peptide, blood coagulation factor or adhesion molecule.

4.   A preparation according to claim 2, wherein the peptide is a hormone.

5.   A preparation according to claim 4, wherein the hormone is a growth hormone.

6.   A preparation according to claim 4, wherein the hormone is an insulin.

7.   A preparation according to claim 2, wherein the peptide is a cytokine.

8.   A preparation according to claim 7, wherein the cytokine is an interferon.

9. A preparation according to claim 2, wherein the peptide is a growth factor.

10. A preparation according to any of claims 1 to 9, wherein the polyvalent metal salt is a transition metal salt.

11. A preparation according to any of claims 1 to 9, wherein the polyvalent metal salt is a zinc salt.

12. A preparation according to any of claims 1 to 11, which contains 1 to 30% (w/w) of the polyvalent metal salt.

13. A preparation according to any of claims 1 to 12, wherein the biodegradable polymer is an aliphatic polyester.

14. A preparation according to claim 13, wherein the aliphatic polyester is a polymer of lactic acid and glycolic acid.

15. A preparation according to claim 14, wherein the composition ratio of lactic acid and glycolic acid is 100/0 to 40/60 (mole%).

16. A preparation according to claim 15, wherein the composition ratio is 90/10 to 45/55 (mole%).

17. A preparation according to claim 14, wherein the weight-average molecular weight of the polymer is 3,000 to 20,000.

18. A preparation according to claim 14, wherein the weight-average molecular weight of the polymer is 3,000 to 14,000.

19. A preparation according to claim 13, wherein the aliphatic polyester is a homopolymer of lactic acid.

20. A preparation according to claim 19, wherein the weight-average molecular weight of the homopolymer is 3,000 to 20,000.

21. A preparation according to claim 19, wherein the weight-average molecular weight of the homopolymer is 3,000 to 14,000.

22. A preparation according to any of claims 1 to 21, wherein the preparation is a microcapsule.

23. A preparation according to claim 22, wherein the microcapsule is for injection.

24. A preparation according to any of claims 1 to 23, which is an injectable one.

25. A preparation according to claim 1, in which the salt is obtainable from a combination of a water-soluble peptide type of physiologically active substance, except for an endothelin antagonist, having acidic group, or a water-soluble salt thereof, with a water-soluble polyvalent metal salt.

26. Use of a water-insoluble or slightly water-soluble polyvalent metal salt of a water-soluble peptide type of physiologically active substance except for an endothelin antagonist and a biodegradable polymer for the production of a sustained-release preparation according to claim 1.

27. A method of producing a sustained-release preparation according to claim 1, which comprises dispersing the water-insoluble or slightly water-soluble polyvalent metal salt of a water-soluble peptide type of physiologically active substance except for an endothelin antagonist in an oil phase containing the biodegradable polymer to make a solid-in-oil emulsion, adding the solid-in-oil emulsion to a water phase to make a solid-in-oil-in-water emulsion, and then in-water drying the solid-in-oil-in-water emulsion.

European Patent Office

# EUROPEAN SEARCH REPORT

Application Number

EP 99 20 3867

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 94 12158 A (ALKERMES CONTROLLED THERAPEUTICS) 9 June 1994 (1994-06-09) * claims 1,2,6 * * page 4, paragraph 2 * * page 9, paragraph 3 - page 10, paragraph 1 * * page 12, paragraph 5 * * page 13; example 1 * | 1-5,9-27 | A61K9/16 A61K9/50 |
| X | WO 93 17668 A (ALKERMES CONTROLLED THERAPEUTICS) 16 September 1993 (1993-09-16) * claims 1-7 * * page 5, paragraph 2 - page 6, paragraph 1 * * page 11, paragraph 2 - page 14, paragraph 3 * | 1-4,9-27 | |

| TECHNICAL FIELDS SEARCHED (Int.Cl.7) |
|---|
| A61K |

The present search report has been drawn up for all claims

| Place of search | Date of completion of the search | Examiner |
|---|---|---|
| THE HAGUE | 30 March 2000 | Ventura Amat, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 99 20 3867

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2000

| Patent document cited in search report | Publication date | Patent family member(s) | | Publication date |
|---|---|---|---|---|
| WO 9412158 A | 09-06-1994 | AU | 670168 B | 04-07-1996 |
| | | AU | 5683594 A | 22-06-1994 |
| | | AU | 694378 B | 16-07-1998 |
| | | AU | 6802996 A | 19-12-1996 |
| | | CA | 2150803 A | 09-06-1994 |
| | | EP | 0674506 A | 04-10-1995 |
| | | JP | 8503950 T | 30-04-1996 |
| | | US | 5891478 A | 06-04-1999 |
| | | US | 5654010 A | 05-08-1997 |
| | | US | 5667808 A | 16-09-1997 |
| WO 9317668 A | 16-09-1993 | AT | 154240 T | 15-06-1997 |
| | | AU | 668384 B | 02-05-1996 |
| | | CA | 2129514 A | 16-09-1993 |
| | | DE | 69311538 D | 17-07-1997 |
| | | EP | 0630234 A | 28-12-1994 |
| | | JP | 7507768 T | 31-08-1995 |
| | | US | 5912015 A | 15-06-1999 |
| | | US | 5656297 A | 12-08-1997 |
| | | US | 5413797 A | 09-05-1995 |